# EUROPEAN PATENT SPECIFICATION

(11) **EP 2 012 682 B1**
(45) Date of publication and mention of the grant of the patent: **25.10.2017**
(21) Application number: 07736227.5
(22) Date of filing: 18.04.2007
(51) Int. Cl.: A61B 17/17, A61B 17/88, A61F 2/46

(54) **TOOLS FOR SPINAL PROSTHESES**
WERKZEUGE FÜR WIRBELSÄULENPROTHESEN
OUTILS POUR PROTHÈSES VERTÉBRALES

(30) Priority: 26.04.2006 US 411029
(43) Date of publication of application: 14.01.2009
(73) Proprietor: Premia Spine Ltd., 42504 Ramat Poleg (IL)
(72) Inventor: ARNIN, Uri, 36031 Kiryat Tivon (IL); KLEIN, Assaf, 38857 Hamaapil (IL)
(74) Representative: Lecomte & Partners
(86) International application number: PCT/IL2007/000487
(87) International publication number: WO 2007/122610

(56) References cited:
- WO-A-2005/089657
- US-A- 4 686 997
- US-A1- 2005 102 028
- US-B2- 6 949 105

## Description

### FIELD OF THE INVENTION

The present invention is generally related to tools and instruments for installation of spinal prostheses.

### BACKGROUND OF THE INVENTION

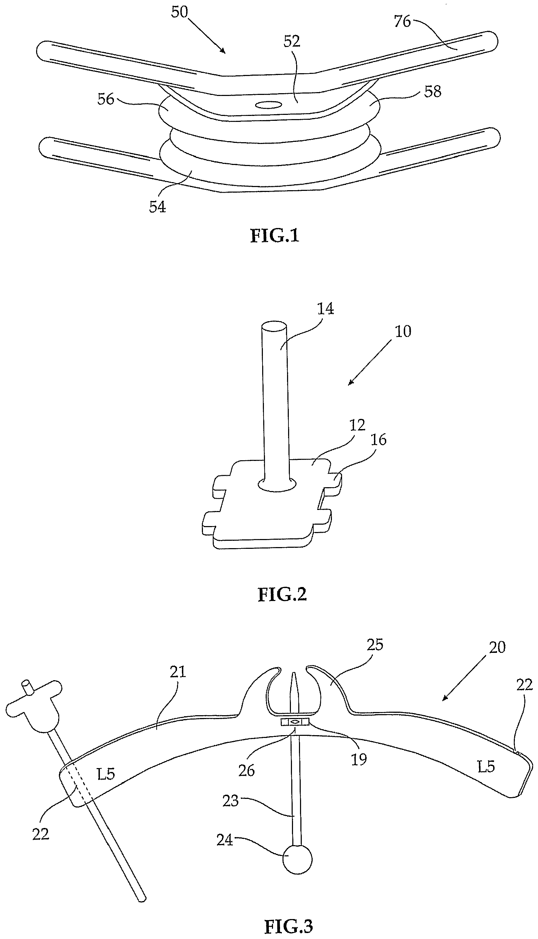

Many kinds of spinal prostheses have been described in the literature. Posterior spinal prostheses are described in US Patent Application Publication 2005/0102028. One of the posterior spinal prostheses described therein is illustrated in Fig. 1, to which reference is now made.

A spinal prosthesis 50 may include an upper (superior) vertebral attachment member 52 and a lower (inferior) vertebral attachment member 54. The attachment members 52 and 54 may be attached to adjacent spinous processes (not shown), or to pedicles (not shown) without having to attach the attachment members 52 and 54 to the spinous processes.

A flexure assembly 56 may be placed between and may be integrally formed with or attached to upper and lower vertebral attachment members 52 and 54. An elastomeric boot 58 may be placed around the flexure assembly 56. The flexure assembly 56 permits flexure of prosthesis 50 about mutually orthogonal axes as well as other directions for omnidirectional flexure in any degree of freedom.

It is noted that the spinal prostheses 50 may be constructed as a unitary body with at least three attachment points attachable to spinal structure. By "unitary body" it is meant that the spinal prosthesis may be attached to the spinal structure as one pre-assembled, contiguous assembly. The surgeon can simply hold the entire unitary body in place during attachment to the spinal structure. This is in contrast to other known prostheses that must be attached to the spinal structure as at least two separate parts, which may or may not articulate with one another. With those prostheses, the surgeon must attach each part separately to some spinal structure and merely "hope" that the parts fit together properly after installment. The unitary construction of the present invention eliminates this problem and greatly facilitates installation of the prosthesis.

One way of installing prosthesis 50 is by means of pedicle screws (not shown), e.g., polyaxial pedicle screws. The swivel heads of the polyaxial pedicle screws may be rotatably attached to rounded prongs 76 jutting from upper and lower vertebral attachment members 52 and 54, such as by means of lock nuts (not shown) that mate with the swivel heads.

In order to install prosthesis 50, a laminectomy may be performed, thereby creating a gap between the spinous processes. The pedicle screws may be screwed into the pedicles. The prosthesis 50 may be inserted in the gap between the spinous processes, with the rounded prongs 76 aligned with the heads of the pedicle screws. The lock nuts may then secure the prosthesis 50 to the pedicle screws.

The configuration of prosthesis 50 may be tailored by the manufacturer to particular vertebrae and to the specific spinal anatomy of a patient. Various design parameters may thus change, such as but not limited to, the angle and length of the prongs 76, size and height of the prosthesis 50, and other factors. Various anatomical parameters may vary, such as but not limited to, configuration and size of the lamina, pedicles, and other spinal anatomical features. It is thus desirable to have a tool or set of tools to facilitate and insure correct installation of prosthesis 50 (or in general any posterior spinal prosthesis).

Patent document published US 6,949,105 B2 discloses a technique and a corresponding trio of devices for determining a target implant location on a skeletal joint. These devices are intended to be pivoted in orthogonal planes. The preamble of claim 1 is based on them. The first one of these devices comprises a bubble level and a marker for marking a transverse center on the vertebral bodies. It has extension arms and a tool receiving portion for receiving the marker. The second one of these devices is intended to be used as reference for positioning a machining fixture like a milling scaffold. The third device is is a tool that holds and instals a screw, said tool having a shaft. This teaching is not designed for aligning pedicle screws.

Patent document published WO 2005/089657 A1 discloses an article with an insertion angle tool for aligning insertion of pedicle screws into spinal structure, which has extension arms that each comprise a tool receiving portion that receives therein a drill, and a level indicator.

### SUMMARY OF THE INVENTION

The present invention seeks to provide tools and instruments for installation of spinal prostheses, as is described more in detail hereinbelow. The tools disclosed herein are particularly advantageous for installing the posterior spinal prostheses shown in Fig. 1, but the invention is not limited to this prosthesis.

There is thus provided in accordance with the present invention an article including an insertion angle tool for aligning insertion of pedicle screws into spinal structure, the insertion angle tool including one or more extension arms that each include a tool receiving portion adapted to receive therein an instrument, and a level indicator, the tool receiving portion being angled such that when the level indicator indicates the insertion angle tool to be level, an instrument held in the tool receiving portion is properly aligned for a particular spinal structure. The tool receiving portion includes a through hole formed through the extension arm. Alternatively, the tool receiving portion may include a bent extremity of the extension arm, or a hooked portion adapted for hooking on to a receiving member of an instrument.

The level indicator may be a pendulum arranged for oscillating between markers, wherein a center mark is provided to indicate the insertion angle tool to be level. Alternatively or additionally, the level indicator may be a bubble vial adapted to indicate when the insertion angle tool is level.

### BRIEF DESCRIPTION OF THE DRAWINGS

In the drawings:
Fig. 1 is a simplified pictorial illustration of a spinal prosthesis of the prior art;
Fig. 2 is a simplified pictorial illustration of a tool used in installing a posterior spinal prosthesis;
Fig. 3 is a simplified pictorial illustration of an insertion angle tool used for properly aligning insertion of pedicle screws prior to installing the posterior spinal prosthesis, constructed and operative in accordance with an embodiment of the present invention;
Fig. 4 is a simplified pictorial illustration of the insertion angle tool of Fig. 3, with a tool receiving portion adapted to hook on to a receiving member mounted on a shaft of a tool, such as a screwdriver, in accordance with another embodiment of the present invention;
Figs. 5A-5D are simplified illustrations of a prosthesis-fastener orientation tool, not being part of the invention, wherein Fig. 5A illustrates placing one of the orientation tools into adjacent pedicle screw heads, Fig. 5B illustrates placing the other one of the orientation tools into adjacent pedicle screw heads, Fig. 5C illustrates that the two orientation tools may be initially misaligned with respect to a reference, and Fig. 5D illustrates that the two orientation tools may be aligned with respect to the reference;
Fig. 6 is a simplified pictorial illustration of a prosthesis-holder tool, not being part of the invention; and
Fig. 7 is a simplified pictorial illustration of another prosthesis-holder tool, not being part of the invention, which may permit using a smaller incision to insert the prosthesis.

### DETAILED DESCRIPTION OF EMBODIMENTS

Reference is now made to Fig. 2, which illustrates a tool 10 used in installing a posterior spinal prosthesis (such as the posterior spinal prosthesis 50 shown in Fig. 1). In preparation, prior to installing the posterior spinal prosthesis, a posterior midline incision may be made in the back of the patient, and as is known in the art, a laminectomy may be performed to create a gap between the spinous processes (e.g., between L4 and L5). Tool 10 may be used to make sure the gap is sized correctly for the type of posterior spinal prosthesis selected (e.g., normal size or low profile). Tool 10 may include a jig or template portion 12 affixed to a handle 14. Template portion 12 may include protrusions 16 that match corresponding portions (e.g., recesses) in the posterior spinal anatomical structure or which may abut against spinal structure so as to help correctly align and seat tool 10 in the spinal structure. The width and size of template portion 12 are chosen to make sure the gap is sized correctly for the type of posterior spinal prosthesis selected.

After the laminectomy has been performed, pedicle screws may be installed in the pedicles. As is known in the art, the pedicle screws may be inserted and screwed into the bone tissue directly or may be inserted by means of a Kirschner wire (K-wire; not shown), the insertion being monitored and guided by x-ray imaging or the like. It is important for the pedicle screws to be inserted at the correct insertion angle for the particular spinal structure (e.g., L5), or at least within a tolerance of the recommended insertion angle, e.g., ±15°, so that the polyaxial head of the pedicle screw can properly compensate for any angular misalignment (the tolerance for the angle between the polyaxial head and the shank of the screw is typically ±20°). It is noted that the invention is not limited to these angular ranges.

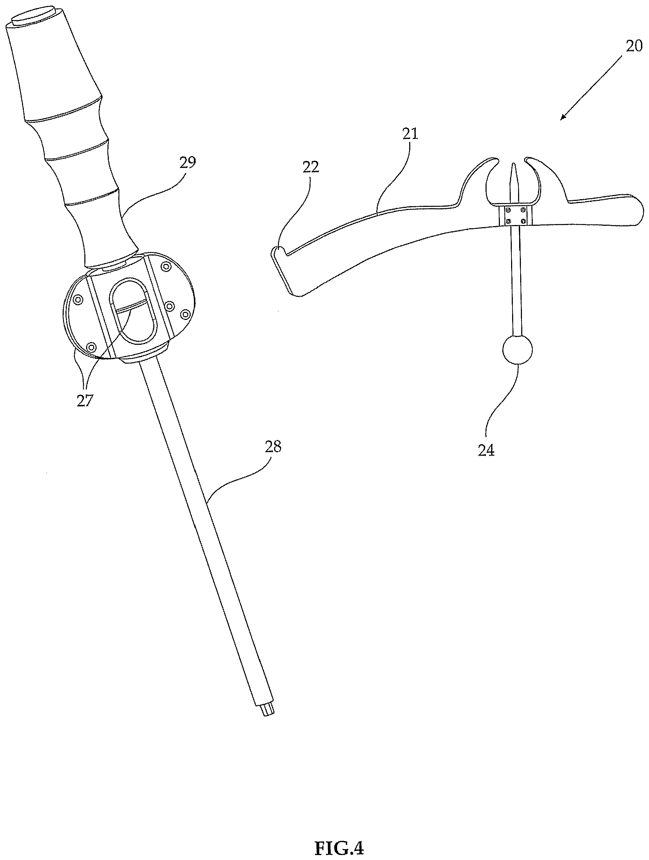

Reference is now made to Fig. 3, which illustrates an insertion angle tool 20 used for properly aligning insertion of pedicle screws prior to installing the posterior spinal prosthesis, constructed and operative in accordance with an embodiment of the present invention. The insertion angle tool includes one or more extension arms 21 that each include a tool receiving portion 22 adapted to receive therein a tool or instrument, such as but not limited to, a drill bit, K-wire, screwdriver, trocar, etc. The tool receiving portion 22 may be a through hole formed through the extension arm 21, or alternatively may be a bent extremity of arm 21 through which the instrument can snugly fit. As another alternative, shown in Fig. 4, tool receiving portion 22 may be a hooked portion that hooks on to a receiving member 27 mounted on a shaft 28 of a tool 29, such as a screwdriver that holds and installs a pedicle screw (not shown in Fig. 4).

The insertion angle tool 20 also includes a level indicator 23, such as but not limited to, a pendulum 24 arranged for oscillating between two markers 25 and is centered at a center mark 26. The tool receiving portion 22 is angled such that when the pendulum 24 is at the center mark 26, the insertion angle tool 20 is level and any instrument held in the tool receiving portion 22 is properly aligned for the particular vertebra. For example, in the illustrated embodiment, when the pendulum 24 is at the center mark 26, any instrument held in the tool receiving portion 22 is properly aligned for the L5 vertebra. The level indicator 23 of insertion angle tool 20 may alternatively or additionally include a bubble level 19 (e.g., a straight bubble vial or a circular omnidirectional bubble vial). After determining the proper insertion angle with insertion angle tool 20, the pedicle screws may be inserted into the spinal structure. The pedicle screws should preferably be left several turns proud of the spinal structure to allow for further alignment with the next tool, which is now described.

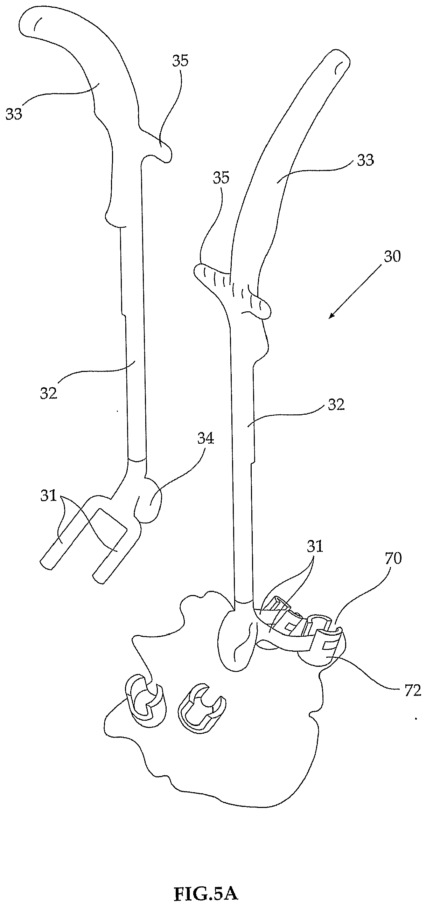

Reference is now made to Figs. 5A-5D, which illustrate a prosthesis-fastener orientation tool 30, used for properly aligning the posterior prosthesis 50 with fasteners that fasten the prosthesis to spinal structure, such as pedicle screws 70 (that may have polyaxial pedicle screw heads 72). The orientation tool 30 may be specific for the particular vertebrae (e.g., L4, L5) to which the prosthesis is being affixed, and may be shaped to take into account a particular lordosis or kyphosis of the patient. In simplistic terms, the orientation tool 30 is a gauge/jig/alignment tool that mimics the attachment of the prosthesis 50 to the pedicle screw heads 72 and ensures the prosthesis 50 is rotationally aligned about a left-right axis with respect to the pedicle screw heads.

The orientation tool 30 may include two identical or near identical tools, each including a structural member, such as prongs 31 that mimic the size, spacing and angular orientation of prongs 76 of prosthesis 50. Prongs 31 are situated at the end of a shaft 32 at the other end of which is a handle 33. Adjacent prongs 31 may be a hook portion 34 for supporting and pivoting orientation tool 30 against spinal structure. An angular gauge 35 may be placed at some point on shaft 32, such as a scale with graduations near handle 33.

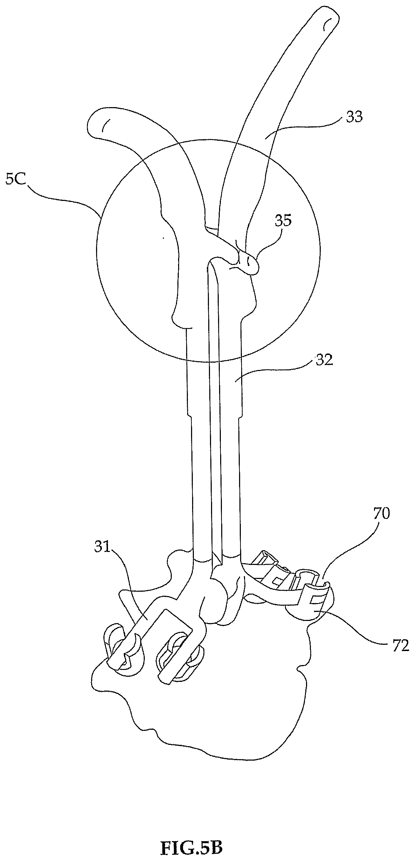

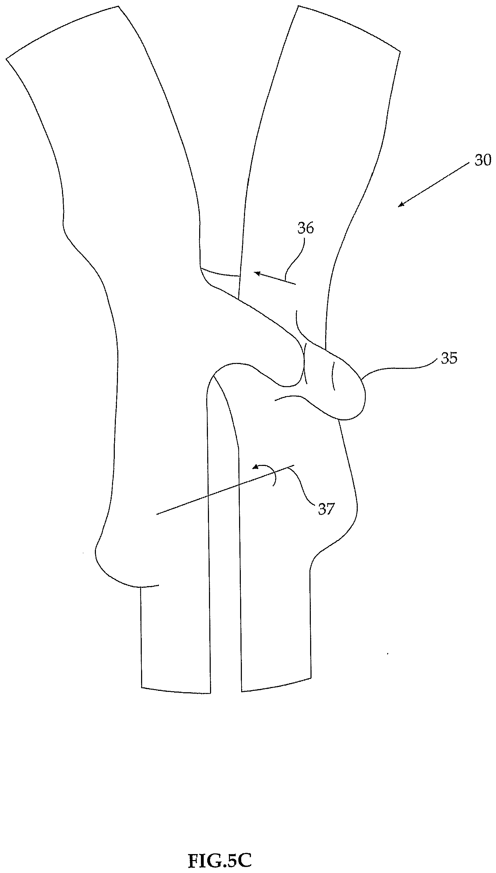

In use, the prongs 31 of one of the orientation tools 30 may be placed into adjacent pedicle screw heads 72, as seen in Fig. 5A, and temporarily fastened to the head 72 with set screws (not shown). In Fig. 5B, the prongs 31 of the other orientation tool 30 may be placed into the other pair of pedicle screw heads 72, and temporarily fastened with set screws (not shown). As seen particularly in Fig. 5C, the two orientation tools 30 may be misaligned in the direction of arrow 36, the misalignment being easily read at angular gauge 35. In other words, the two orientation tools 30 are rotationally misaligned about a left-right axis 37 in the sagittal plane.

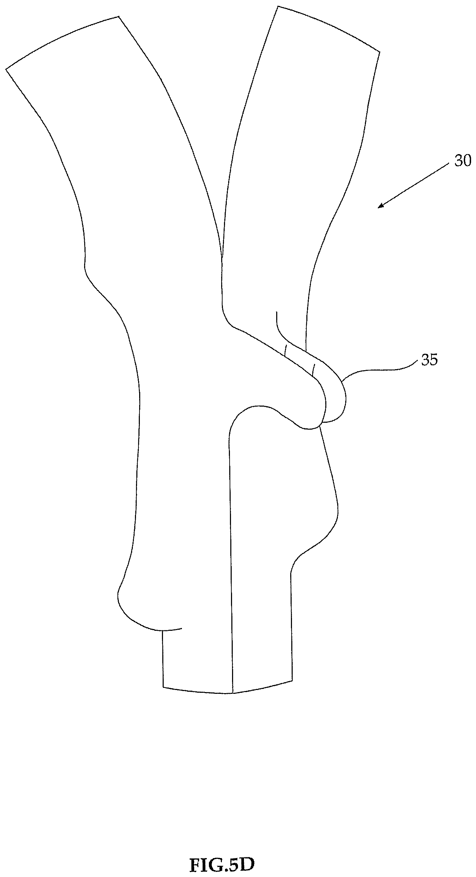

Aligning the two orientation tools 30 may be easily accomplished by removing one of the orientation tools 30 from its pedicle screw heads 72 and further screwing one or both of the pedicle screws 70 further into the bone. This changes and fine-tunes the angular orientation until upon re-attachment of the orientation tool 30 to the pedicle screws 70, the two orientation tools 30 are exactly aligned as seen in Fig. 5D. After removal of the two orientation tools 30 and the temporary set screws, the prosthesis 50 may be attached to the pedicle screw heads 72.

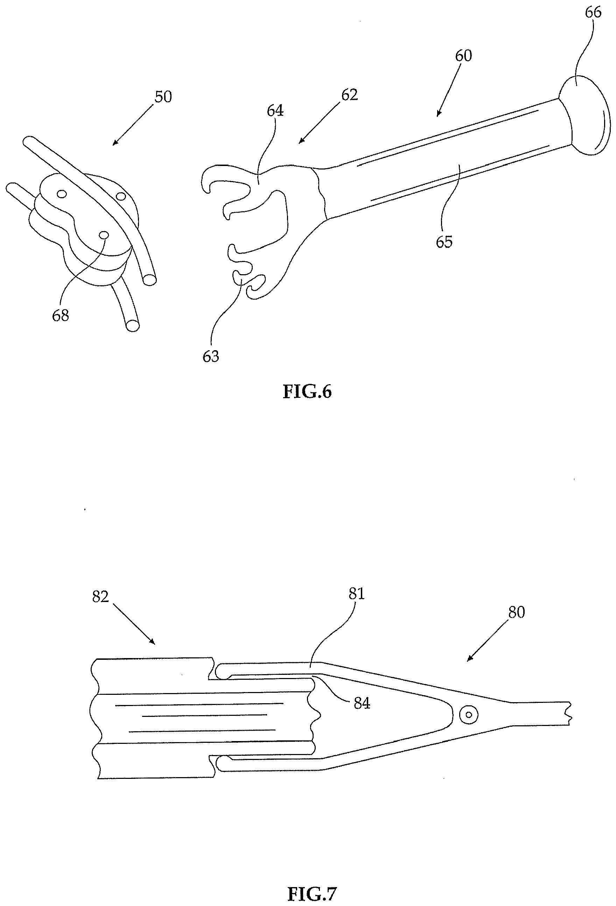

Reference is now made to Fig. 6, which illustrates a prosthesis-holder tool 60. Tool 60 may be used to grasp prosthesis 50 and insert it through the incision for attachment to the pedicle screw heads 72 (not shown in Fig. 6). Tool 60 may include a grasping portion 62 that includes a pair of jaws 64 rotatingly mounted on a handle 65. Handle 65 may comprise an actuator (e.g., rotatable knob) 66 that may be turned to open or close the jaws 64. The jaws 64 may include one or more teeth 63, which may be received in holding structure found in prosthesis 50 (e.g., one or more screw heads or holes 68).

Reference is now made to Fig. 7, which illustrates another prosthesis-holder tool 80. Tool 80 may be used to grasp a prosthesis 82 for insertion through an incision and installation in the body. Tool 80 differs from tool 60 in that tool 80 has jaws 81 constructed to grasp a recess 84 formed in prosthesis 82 (which may be similar to or different from prosthesis 50) such that jaws 81 are flush or below the surface of prosthesis 82. This provides a low profile insertion tool and may permit using a smaller incision to insert the prosthesis 82.

## Claims

1. An article comprising:
a tool (29) that holds and installs a pedicle screw (70), said tool (29) having a shaft (28);
an insertion angle tool (20) for aligning insertion of said pedicle screw (70) into spinal structure, said insertion angle tool (20) comprising one or more extension arms (21), and a level indicator (23), **characterized in that** said one or more extension arms each comprise a tool receiving portion (22) that receives therein said tool, said tool receiving portion (22) is angled such that when said level indicator (23) indicates said insertion angle tool (20) to be level, the tool (29) held in said tool receiving portion (22) is properly aligned to insert the pedicle screw (70) into a particular vertebra, and said tool receiving portion (22) comprises a through hole formed through said extension arm (21), or a bent extremity of said extension arm (21) through which the tool (29) can snugly fit, or a hooked portion that hooks on to a receiving member (27) mounted on the shaft (28) of the tool (29) that holds and installs a pedicle screw.

2. The article according to claim 1, wherein said level indicator (23) comprises a pendulum (24) arranged for oscillating between markers (25), and wherein a center mark (26) is provided to indicate said insertion angle tool to be level.

3. The article according to claim 1, wherein said level indicator comprises a bubble vial adapted to indicate when said insertion angle tool is level.

## Patentansprüche

1. Artikel, umfassend:
ein Werkzeug (29), das eine Pedikelschraube (70) festhält und installiert, wobei das Werkzeug (29) einen Schaft (28) aufweiset; ein Einfügewinkelwerkzeug (20) zur Ausrichtung des Einfügens der Pedikelschraube (70) in Spinalstruktur, wobei das Einfügewinkelwerkzeug (20) einen oder mehrere Verlängerungsarme (21) und eine Niveauanzeige (23) umfasst, **dadurch gekennzeichnet, dass** der eine oder die mehreren Verlängerungsarme jeder ein Werkzeugaufnahmeteil (22) umfassen, welches das Werkzeug darin aufnimmt, wobei das Werkzeugaufnahmeteil (22) so angewickelt ist, dass, wenn die Niveauanzeige (23) anzeigt, dass das Einfügewinkelwerkzeug (20) waagerecht ist, das in dem Werkzeugaufnahmeteil (22) festgehaltene Werkzeug (29) richtig ausgerichtet ist, um die Pedikelschraube (70) in einen bestimmten Wirbel einzufügen, und das Werkzeugaufnahmeteil (22) ein durch den Verlängerungsarm (21) hindurch gebildetes Durchgangsloch, oder ein gebogenes Ende des Verlängerungsarms (21), durch welches das Werkzeug (29) passgenau passen kann, oder ein hakenförmiges Teil, das an einem an dem Schaft (28) des Werkzeugs (29), das eine Pedikelschraube festhält und installiert, montierten Aufnahmeelement (27) einhakt, aufweist.

2. Artikel nach Anspruch 1, wobei die Niveauanzeige (23) ein Pendel (24) umfasst, das zum Oszillieren zwischen Markierungselementen (25) eingerichtet ist, und wobei eine zentrale Markierung (26) vorgesehen ist, um anzuzeigen, dass das Einfügewinkelwerkzeug waagerecht ist.

3. Artikel nach Anspruch 1, wobei die Niveauanzeige eine Röhrenlibelle umfasst, die dazu eingerichtet ist, anzuzeigen, wenn das Einfügewinkelwerkzeug waagerecht ist.

## Revendications

1. Article comprenant :
un outil (29) qui maintient une vis pédiculaire (70) et sert au montage de cette dernière, ledit outil (29) possédant une tige (28) ;
un outil d'angle d'insertion (20) pour l'alignement d'insertion de ladite vis pédiculaire (70) dans la structure spinale, ledit outil d'angle d'insertion (20) comprenant un ou plusieurs bras d'extension ; et
un indicateur de niveau (23), **caractérisé en ce que** lesdits un ou plusieurs bras d'extension comprennent chacun une portion de réception d'outil (22) dans laquelle vient se loger ledit outil, ladite portion de réception d'outil (22) formant un angle tel que, lorsque ledit indicateur de niveau (23) indique la mise à niveau dudit outil d'angle d'insertion (20), l'outil (29) maintenu dans ladite portion de réception d'outil (22) est correctement aligné pour insérer la vis pédiculaire (70) dans une vertèbre particulière, et ladite portion de réception d'outil (22) comprend un trou de passage formé à travers ledit bras d'extension (21) ou une extrémité coudée dudit bras d'extension (21) par laquelle l'outil (29) peut venir se disposer en ajustage serré, ou une portion en forme de crochet qui s'accroche à un élément de réception (27) monté sur la tige (28) de l'outil (29) qui maintient une vis pédiculaire et sert de montage à celle-ci.

2. Article selon la revendication 1, dans lequel ledit indicateur de niveau (23) comprend un pendule (24) prévu pour osciller entre des marqueurs (25) et dans lequel est prévue une marque centrale (26) pour indiquer la mise à niveau dudit angle d'insertion.

3. Article selon la revendication 1, dans lequel ledit indicateur de niveau comprend une ampoule à bulle conçue pour indiquer la mise à niveau dudit angle d'insertion.
